# FASCICULE DE BREVET EUROPEEN

(11) **EP 1 415 641 B1**
(45) Date de publication et mention de la délivrance du brevet: **20.12.2006**
(21) Numéro de dépôt: 03292579.4
(22) Date de dépôt: 16.10.2003
(51) Int. Cl.: A61K 8/06, A61K 8/34, A61K 8/81, A61Q 5/04, A61Q 5/10

(54) **Emulsion crème oxydante, huile-dans-eau, pour le traitement des fibres kératiniques humaines**
Oxidationscreme in Form einer Öl-in-Wasser Emulsion zur Behandlung menschlicher Keratinfasern
Oxidating creme in form of an oil-in-water emulsion for treating human keratin fibres

(30) Priorité: 23.10.2002 FR 0213240
(43) Date de publication de la demande: 06.05.2004
(73) Titulaire: L'ORÉAL, 75008 Paris (FR)
(72) Inventeur: Legrand, Frédéric, 92400 Courbevoie (FR)
(74) Mandataire: Wattremez, Catherine

(56) Documents cités:
- FR-A- 1 264 797
- FR-A- 2 818 543

## Description

La présente demande concerne une émulsion crème oxydante destinée au traitement des fibres kératiniques humaines et plus particulièrement des cheveux, comprenant au moins un agent oxydant, au moins un alcool gras en (C₈-C₃₀), au moins un agent tensioactif non ionique et/ou anionique et au moins un polymère amphiphile comportant au moins un motif acide 2-acrylamido-2-méthylpropane-sulfonique sous forme libre ou partiellement ou totalement neutralisée et au moins un motif hydrophobe comportant de 6 à 50 atomes de carbone.
La demande concerne aussi l'utilisation dudit polymère amphiphile pour stabiliser la viscosité d'une émulsion oxydante huile-dans-eau comprenant au moins un alcool gras en (C₈-C₃₀) et au moins un agent tensioactif non ionique et/ou anionique.
Elle concerne également les procédés de teinture, de décoloration, et de déformation permanente mettant en oeuvre ladite émulsion.

En cosmétique, dans les domaines de la teinture, de la décoloration et de la déformation permanente des fibres kératiniques humaines et plus particulièrement des cheveux, on utilise des compositions oxydantes.
Ainsi, en teinture d'oxydation des cheveux, des compositions oxydantes sont mélangées aux colorants d'oxydation (bases et coupleurs), qui sont incolores par eux-mêmes, pour engendrer des composés colorés et colorants par un processus de condensation oxydative. Des compositions oxydantes sont également utilisées en teinture directe des cheveux en mélange avec certains colorants directs qui sont colorés et colorants pour obtenir une coloration avec un effet éclaircissant des cheveux. Parmi les agents oxydants classiquement utilisés pour la teinture des fibres kératiniques, on peut citer le peroxyde d'hydrogène ou des composés susceptibles de produire le peroxyde d'hydrogène par hydrolyse tels que le peroxyde d'urée, les persels comme les perborates et persulfates, le peroxyde d'hydrogène étant plus particulièrement préféré.

En décoloration des cheveux, les compositions de décoloration contiennent un ou plusieurs agents oxydants. Parmi ces agents oxydants, les plus classiquement utilisés sont le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.
Ces compositions sont formées principalement de produits anhydres qui contiennent des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.
Les compositions de décoloration se présentent également sous forme d'émulsions huile-dans-eau à base de peroxyde d'hydrogène, prêtes à l'emploi.
Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

En déformation permanente des cheveux, dans un premier temps, on réalise l'ouverture des liaisons disulfures -S-S- de la kératine (cystine) à l'aide d'une composition contenant un agent réducteur adapté (étape de réduction) puis, après avoir rincé la chevelure ainsi traitée, on reconstitue dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en pli.
Les compositions oxydantes nécessaires à la mise en oeuvre de l'étape de fixation, sont le plus souvent des compositions à base d'eau oxygénée.

Dans ces applications cosmétique (voir par exemple FR-1 264 797), les compositions oxydantes sont souvent formulées sous forme d'émulsions huile-dans-eau (H/E) à base d'alcool gras et de tensioactif non ionique ou anionique, afin d'optimiser les qualités d'application et d'usage des compositions de teinture, de décoloration ou de déformation permanente qui les comprennent, et notamment, afin qu'elles aient une consistance suffisante pour ne pas couler hors des zones de la chevelure ou des mèches de cheveux à traiter.
On a constaté cependant que les émulsions oxydantes H/E formées à partir d'alcool(s) gras et de tensioactif(s) non ionique(s) ou anionique(s) évoluaient beaucoup en texture et viscosité dans le temps, et que les compositions cosmétiques qui en sont formées perdaient en qualité d'usage. Par exemple, de telles compositions peuvent s'épaissir fortement dans le temps, rendant ainsi progressivement très difficile leur restitution du dispositif les contenant par les professionnels de la coiffure.

La demanderesse a découvert de manière surprenante qu'il est possible d'obtenir des émulsions crèmes oxydantes H/E dont la viscosité et la texture évoluent significativement moins dans le temps si on introduit dans l'émulsion oxydante H/E un polymère amphiphile comportant au moins un motif acide 2-acrylamido-2-méthylpropane-sulfonique (A.M.P.S.) sous forme libre ou partiellement ou totalement neutralisée et au moins un motif hydrophobe comportant de 6 à 50 atomes de carbone.
Ainsi, les compositions de teinture, de décoloration ou de déformation permanente qui les comprennent sont stables et présentent des qualités d'application et d'usage améliorées et plus performantes.

Cette découverte est à la base de la présente invention.

La présente invention a donc pour objet une émulsion crème oxydante, huile-dans-eau, destinée au traitement des matières kératiniques, caractérisée par le fait qu'elle comprend au moins un agent oxydant, au moins un alcool gras en (C₈-C₃₀), au moins un agent tensioactif non ionique et/ou anionique et au moins un polymère amphiphile comportant au moins un motif acide 2-acrylamido-2-méthylpropane-sulfonique sous forme libre ou partiellement ou totalement neutralisée de formule (I) suivante et au moins un motif hydrophobe comportant de 6 à 50 atomes de carbone, 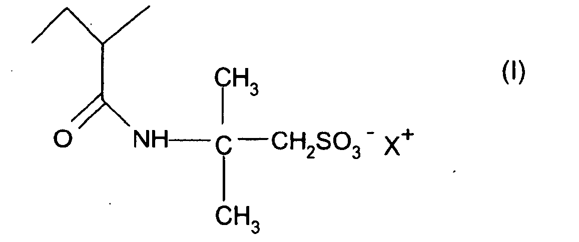 dans laquelle X⁺ est un proton, un cation de métal alcalin, un cation alcalino-terreux ou l'ion ammonium.

L'invention concerne également l'utilisation dudit polymère pour stabiliser la viscosité d'une émulsion oxydante H/E comprenant au moins un alcool gras et au moins un agent tensioactif non ionique et/ou anionique.

Mais d'autres caractéristiques, aspects, objets et avantages de l'invention apparaîtront encore plus clairement à la lecture de la description et des exemples qui suivent.

### Polymères amphiphiles comportant au moins un motif acide 2-acrylamido-2-méthylpropane-sulfonique (A.M.P.S.) sous forme libre ou partiellement ou totalement neutralisée et au moins un motif hydrophobe comportant de 6 à 50 atomes de carbone.

On entend par polymère amphiphile, tout polymère comportant à la fois une partie hydrophile et une partie hydrophobe et notamment une chaîne grasse.
La partie hydrophobe présente dans les polymères de l'invention comporte de préférence de 12 à 22 atomes de carbone.

Les polymères amphiphiles conformes à l'invention ont généralement un poids moléculaire moyen en nombre allant de 1000 à 20 000 000 g/mole, de préférence allant de 20 000 à 5 000 000 et plus préférentiellement encore de 100 000 à 1 500 000 g/mole.

Les polymères amphiphiles selon l'invention peuvent être réticulés ou non-réticulés.

De préférence, on choisit des polymères amphiphiles réticulés.

Lorsqu'ils sont réticulés, les agents de réticulation peuvent être choisis parmi les composés à polyinsaturation oléfinique couramment utilisés pour la réticulation des polymères obtenus par polymérisation radicalaire.

On peut citer par exemple, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

On utilisera plus particulièrement le méthylène-bis-acrylamide, le méthacrylate d'allyle ou le triméthylol propane triacrylate (TMPTA). Le taux de réticulation variera en général de 0,01 à 10% en mole et plus particulièrement de 0,2 à 2% en mole par rapport au polymère.

Les polymères amphiphiles conformes à l'invention peuvent notamment être choisis parmi les polymères amphiphiles statistiques d'AMPS modifiés par réaction avec une n-monoalkylamine ou une di-n-alkylamine en C₆-C₂₂, et tels que ceux décrits dans la demande de brevet WO00/31154. Ces polymères peuvent également contenir d'autres motifs hydrophiles choisis par exemple parmi les acides (méth)acryliques, leurs dérivés alkyl substitués en β ou leurs esters obtenus avec des monoalcools ou des mono- ou poly- alkylèneglycols, les (méth)acrylamides, la vinylpyrrolidone, l'anhydride maléique, l'acide itaconique ou l'acide maléique ou les mélanges de ces composés.

Ces mêmes copolymères peuvent contenir en outre un ou plusieurs motifs ne comportant pas de chaîne grasse tels que les acides (méth)acryliques, leurs dérivés alkyl substitués en β ou leurs esters obtenus avec des monoalcools ou des mono- ou poly-alkylèneglycols, les (méth)acrylamides, la vinylpyrrolidone, l'anhydride maléique, l'acide itaconique ou l'acide maléique ou les mélanges de ces composés.

Ces copolymères sont décrits notamment dans la demande de brevet EP-A-750899, le brevet US 5089578 et dans les publications de Yotaro Morishima suivantes :
- « Self-assembling amphiphilic polyelectrolytes and their nanostructures - Chinese Journal of Polymer Science Vol. 18, N°40, (2000), 323-336. »
- « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » ;
- « Solution properties of miscelle networks formed by non-ionic moieties covalently bound to an polyelectrolyte : salt effects on rheological behavior - Langmuir, 2000, Vol.16, N°12, 5324-5332 » ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Les motifs hydrophobes de ces copolymères particuliers sont choisis de préférence parmi les acrylates ou les acrylamides de formule (II) suivante : 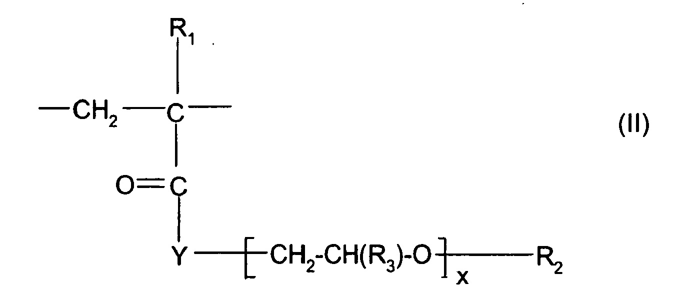 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant de 6 à 50 atomes de carbone et plus préférentiellement de 12 à 22 atomes de carbone; x désigne un nombre de moles d'oxyde d'alkylène et varie de 0 à 100.

Le radical R₂ est choisi de préférence parmi les radicaux alkyles en C₆-C₁₈ linéaires (par exemple n-hexyle, n-octyle, n-décyle, n-hexadécyle, n-dodécyle), ramifiés ou cycliques (par exemple cyclododécane (C₁₂) ou adamantane (C₁₀)) ; les radicaux alkylperfluorés en C₆-C₁₈(par exemple le groupement de formule -(CH₂)₂-(CF₂)₉-CF₃) ; le radical cholestéryle (C₂₇) ou un reste d'ester de cholestérol comme le groupe oxyhexanoate de cholestéryle ; les groupes polycycliques aromatiques comme le naphtalène ou le pyrène. Selon une forme particulièrement préférée de l'invention, le motif de formule (II) comporte au moins un motif oxyde d'alkylène (x ≥1) et de préférence une chaîne polyoxyalkylénée. La chaîne polyoxyalkylénée, de façon préférentielle, est constituée de motifs oxyde d'éthylène et/ou de motifs oxyde de propylène et encore plus particulièrement constituée de motifs oxyde d'éthylène. Le nombre de motifs oxyalkylénés varie en général de 3 à 100 et plus préférentiellement de 3 à 50 et encore plus préférentiellement de 7 à 25.

Parmi ces polymères, on peut citer :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(méth)acrylate par rapport au polymère, tels que ceux décrits dans la demande EP-A750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, tels que ceux décrits dans le brevet US- 5089578.

On peut également citer les copolymères non-réticulés et réticulés d'AMPS partiellement ou totalement neutralisé et de méthacrylate de dodécyle ainsi que les copolymères non-réticulés et réticulés d'AMPS partiellement ou totalement neutralisé et de n-dodécylméthacrylamide, tels que ceux décrits dans les articles de Morishima cités ci-dessus.

On citera plus particulièrement les copolymères constitués de motifs acide 2-acrylamido-2-méthylpropane-sulfonique (AMPS) de formule (I) et de motifs de formule (III) suivante : 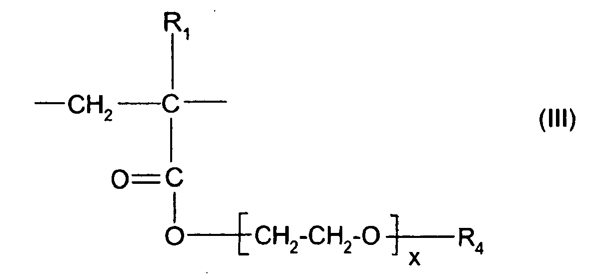 dans laquelle x désigne un nombre entier variant de 3 à 100, de préférence de 5 à 80 et plus préférentiellement de 7 à 25 ; R₁ a la même signification que celle indiquée ci-dessus dans la formule (II) et R₄ désigne un alkyle linéaire ou ramifié en C₆-C₂₂ et plus préférentiellement en C₁₀-C₂₂.

Les polymères particulièrement préférés sont ceux pour lesquels dans le motif (III), x = 25, R₁ désigne méthyle et R₄ représente C₁₆-C₁₈ ou C₂₂.

Les polymères amphiphiles préférés conformes à l'invention peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le ABAH (2,2-azobis-[2-amidinopropane] hydrochloride), les peroxydes organiques tels que le peroxyde de dilauryle, le peroxyde de benzoyle, l'hydroperoxyde de tert-butyle, etc..., des composés peroxydés minéraux tels que le persulfate de potassium ou d'ammonium, ou H₂O₂ éventuellement en présence de réducteurs.

Ils sont notamment obtenus par polymérisation radicalaire en milieu tert-butanol dans lequel ils précipitent.

En utilisant la polymérisation par précipitation dans le tert-butanol, il est possible d'obtenir une distribution de la taille des particules du polymère particulièrement favorable pour ses utilisations.

La distribution de la taille des particules du polymère peut être déterminée par exemple par diffraction laser ou analyse d'image.

Une distribution intéressante pour de type de polymère et déterminée par analyse d'image est la suivante: 60,2% inférieur à 423 microns, 52,0% inférieur à 212 microns, 26,6% inférieur à 106 microns, 2,6% inférieur à 45 microns et 26,6% supérieur à 850 microns.

La réaction peut être conduite à une température comprise entre 0 et 150°C, de préférence entre 10 et 100°C , soit à pression atmosphérique, soit sous pression réduite. Elle peut aussi être réalisée sous atmosphère inerte, et de préférence sous azote.

Selon ce procédé, on a notamment polymérisé l'acide 2-acrylamido-2-méthylpropane-sulfonique (AMPS) ou l'un de ses sels de sodium ou d'ammonium, avec un ester de l'acide (méth)acrylique et,
- d'un alcool en C₁₀-C₁₈ oxyéthyléné par 8 moles d'oxyde d'éthylène (GENAPOL® C-080 de la société HOECHST/CLARIANT),
- d'un alcool oxo en C₁₁ oxyéthyléné par 8 moles d'oxyde d'éthylène (GENAPOL® UD-080 de la société HOECHST/CLARIANT),
- d'un alcool oxo en C₁₁ oxyéthyléné par 7 moles d'oxyde d'éthylène (GENAPOL® UD-070 de la société HOECHST/CLARIANT),
- d'un alcool en C₁₂-C₁₄ oxyéthyléné par 7 moles d'oxyde d'éthylène (GENAPOL® LA-070 de la société HOECHST/CLARIANT),
- d'un alcool en C₁₂-C₁₄ oxyéthyléné par 9 moles d'oxyde d'éthylène (GENAPOL® LA-090 de la société HOECHST/CLARIANT),
- d'un alcool en C₁₂-C₁₄ oxyéthyléné par 11 moles d'oxyde d'éthylène (GENAPOL® LA-110 de la société HOECHST/CLARIANT),
- d'un alcool en C₁₆-C₁₈ oxyéthyléné par 8 moles d'oxyde d'éthylène (GENAPOL® T-080 de la société HOECHST/CLARIANT),
- d'un alcool en C₁₆-C₁₈ oxyéthyléné par 15 moles d'oxyde d'éthylène (GENAPOL® T-150 de la société HOECHST/CLARIANT),
- d'un alcool en C₁₆-C₁₈ oxyéthyléné par 11 moles d'oxyde d'éthylène (GENAPOL® T-110 de la société HOECHST/CLARIANT),
- d'un alcool en C₁₆-C₁₈ oxyéthyléné par 20 moles d'oxyde d'éthylène (GENAPOL® T-200 de la société HOECHST/CLARIANT),
- d'un alcool en C₁₆-C₁₈ oxyéthyléné par 25 moles d'oxyde d'éthylène (GENAPOL® T-250 de la société HOECHST/CLARIANT),
- d'un alcool en C₁₈-C₂₂ oxyéthyléné par 25 moles d'oxyde d'éthylène et/ou d'un alcool iso C₁₆-C₁₈ oxyéthyléné par 25 moles d'oxyde d'éthylène.

Ces copolymères d'A.M.P.S. et leur procédé de préparation ont notamment été décrits dans la demande de brevet français N° 2 818 543.

La concentration molaire en % des motifs de formule (II) et des motifs de formule (III) dans les polymères selon l'invention variera en fonction de l'application cosmétique souhaitée et des propriétés rhéologiques de la formulation recherchées. Elle peut varier entre 0,1 et 99,9% en moles.

De préférence pour les polymères les plus hydrophobes, la proportion molaire en motifs de formule (II) ou (III) varie de 50,1 à 99,9%, plus particulièrement de 70 à 95% et encore plus particulièrement de 80 à 90%.

De préférence pour les polymères peu hydrophobes, la proportion molaire en motifs de formule (II) ou (III) varie de 0,1 à 50%, plus particulièrement de 5 à 25% et encore plus particulièrement de 10 à 20%.

La distribution des monomères dans les polymères de l'invention peut être, par exemple, alternée, bloc (y compris multibloc) ou quelconque.

Les viscosités (mesurées à 25°C au viscosimètre Brookfield aiguille 7) des solutions aqueuses à 1% vont de préférence de 20 000 mPas à 100 000 mPas et plus particulièrement de 60 000 mPas à 70 000 mPas.

Les polymères amphiphiles conformes à l'invention sont présents dans les émulsions oxydantes H/E dans des concentrations allant de 0,01 à 10% en poids, plus préférentiellement de 0,01 à 5%, encore plus préférentiellement de 0,01 à 2% en poids, par rapport au poids total de l'émulsion.

### Agent oxydant

Selon l'invention, l'agent oxydant est de préférence choisi dans le groupe formé par le peroxyde d'hydrogène et les composés susceptibles de produire par hydrolyse du peroxyde d'hydrogène, ou leurs mélanges.
L'agent oxydant est ainsi choisi de préférence dans le groupe formé par l'eau oxygénée, le peroxyde d'urée, les bromates ou ferricyanures de métaux alcalins, les persels tels que les perborates ou les persulfates ou leurs mélanges. On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Plus particulièrement, l'agent oxydant est le peroxyde d'hydrogène, et notamment l'eau oxygénée.

La concentration en peroxyde d'hydrogène peut varier de 0,15 à 12%, de préférence de 0,6 à 9% et celle en composé susceptible de former du peroxyde d'hydrogène par hydrolyse de 0,1 à 25 % en poids par rapport au poids total de l'émulsion oxydante.

De préférence, lorsque l'agent oxydant est de l'eau oxygénée, l'émulsion oxydante selon l'invention contient au moins un agent stabilisant de l'eau oxygénée qui peut notamment être choisi parmi les pyrophosphates des métaux alcalins ou alcalino-terreux, les stannates des métaux alcalins ou alcalino-terreux, la phénacétine ou les sels d'acides et d'oxyquinoléine, comme le sulfate d'oxyquinoléine. De manière avantageuse, on utilise au moins un stannate en association ou non à au moins un pyrophosphate.
On peut également utiliser l'acide salicylique et ses sels, l'acide pyridine dicarboxylique et ses sels, le paracétamol et les systèmes constitués a) d'un tampon [borate de métal alcalin (Na, K) ou d'ammonium et de préférence tétraborate de Na, décahydraté], b) d'un agent alcalin (NH₄OH monoéthanolamine, carbonate d'ammonium, carbonate acide d'ammonium, soude), et c) d'un agent séquestrant d'ion métallique lourd (Fe, Mn, Cu) tels que ceux décrits dans les demandes de brevets WO-01/72271, WO-01/72272, WO-01/52801.

Dans les émulsions oxydantes selon l'invention, la concentration en agents stabilisants de l'eau oxygénée peut varier de 0,0001 % à 5% en poids et de préférence de 0,01 à 2% en poids par rapport au poids total des émulsions oxydantes.
Dans les émulsions oxydantes selon l'invention à l'eau oxygénée, le rapport des concentrations du peroxyde d'hydrogène aux agents stabilisants peut varier de 0,05 à 1000 et de préférence de 0,1 à 500 et plus préférentiellement encore de 1 à 200. De même, le rapport des concentrations du ou des polymère(s) amphiphile(s) selon l'invention aux agents stabilisants peut varier de 0,05 à 1000 et de préférence de 0,1 à 500 et plus préférentiellement encore de 1 à 200.
De préférence, selon l'invention, le rapport des concentrations du ou des polymère(s) amphiphile(s) selon l'invention aux agents oxydants est compris entre 0,001 et 10, les quantités desdits polymères et oxydants étant exprimées en matières actives (peroxyde d'hydrogène pour l'eau oxygénée). Plus préférentiellement encore, ce rapport est compris entre 0,01 et 5 et plus particulièrement encore entre 0,02 et 1.

### Alcools gras en C₈-C₃₀

Par alcool gras selon l'invention, on entend tout alcool gras pur, saturé ou insaturé, linéaire ou ramifié.
Parmi ces alcools gras on préfère ceux en C₁₂-C₂₂.
On peut citer parmi eux, les alcools laurique, cétylique, stéarylique, oléïque, béhénique, linoléïque, undécylénique, palmitoléïque, linolénique, arachidonique, érucique, et leurs mélanges.
L'alcool cétylique est plus particulièrement préféré.
Dans les émulsions oxydantes selon l'invention, la concentration en alcools gras peut varier d'environ 0,1 à 30% en poids et plus préférentiellement d'environ 0,5 à 15% en poids par rapport au poids total de l'émulsion.

### Agents tensioactifs non ioniques et/ou anioniques

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les alcools gras glycérolés; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.
Selon l'invention, parmi les agents tensioactifs non ioniques on préfère utiliser les alcools gras glycérolés. Les alcools gras glycérolés présentent notamment la structure suivante : 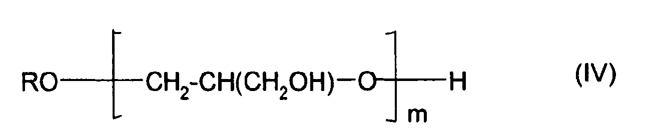 dans laquelle :
R représente un radical saturé ou insaturé, linéaire ou ramifié, comportant de 8 à 40 atomes de carbone et de préférence de 10 à 30 atomes de carbone ;
m représente un nombre allant de 1 à 30 et de préférence de 1 à 10.

Comme composés de ce type, on peut citer, l'alcool laurique à 4 moles de glycérol (nom INCl : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.
L'alcool gras peut représenter un mélange d'alcools gras au même titre que la valeur de m représente une valeur statistique, ce qui signifie que dans un produit commercial peuvent coexister plusieurs espèces d'alcools gras polyglycérolés sous forme d'un mélange.

Les agents tensioactifs anioniques utilisables, seuls ou mélanges, dans le cadre de la présente invention, sont notamment (liste non limitative) les sels (en particulier sels alcalins, et notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Selon l'invention, parmi les agents tensioactifs anioniques on préfère utiliser les alkylsulfates, les alkyléthersulfates et les α-oléfine-sulfonates.

Plus préférentiellement selon l'invention, on utilise des mélanges de tensioactifs non ioniques et anioniques.

Les quantités d'agents tensioactifs non ioniques et/ou anioniques présents dans l'émulsion selon l'invention peuvent varier d'environ 0,1 à 30% et de préférence d'environ 0,5 à 15% du poids total de l'émulsion.

Les émulsions crèmes oxydantes H/E selon l'invention présentent un pH variant de préférence de 1 à 6, et plus préférentiellement de 2 à 4.
Le pH de ces émulsions selon l'invention peut être obtenu et/ou ajusté classiquement par ajout soit d'agents basifiants, tels que par exemple l'ammoniaque, la monoéthanolamine, la diéthanolamine, la triéthanolamine, l'isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide phosphorique, l'acide acétique, l'acide lactique, l'acide citrique, l'acide tartrique et l'acide borique.

L'émulsion crème oxydante H/E peut encore comprendre une quantité efficace d'additifs connus pour leur utilisation dans les compositions oxydantes pour la teinture des cheveux par oxydation, pour la décoloration et pour la déformation permanente des cheveux tels que des agents conservateurs, des agents séquestrants tel que l'EDTA, le DTPA et l'acide étidronique, des agents antimousse comme le siméthicone, des polymères substantifs cationiques et amphotères, des polymères épaississants hydrosolubles, des parfums et des colorants pour colorer l'émulsion.

Bien entendu, l'homme de l'art veillera à choisir le ou les éventuels composés complémentaires mentionnés ci-avant, de manière telle que les propriétés avantageuses attachées intrinsèquement à l'émulsion selon l'invention ne soient pas, ou substantiellement pas, altérées par la ou les adjonctions envisagées.

Un autre objet de l'invention est un procédé de teinture d'oxydation des fibres kératiniques humaines et en particulier des cheveux mettant en oeuvre une composition colorante comprenant dans un support approprié pour la teinture desdites fibres au moins un colorant d'oxydation et une émulsion oxydante telle que définie ci-dessus.
Selon ce procédé, on applique sur les fibres ladite composition colorante, la couleur étant révélée à pH acide, neutre ou alcalin à l'aide d'une émulsion oxydante selon l'invention qui est appliquée simultanément ou séquentiellement, avec ou sans rinçage intermédiaire. Selon une forme de mise en oeuvre particulièrement préférée du procédé de teinture selon l'invention, on mélange, au moment de l'emploi, la composition colorante avec une émulsion oxydante selon l'invention. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

La composition oxydante selon l'invention peut également être utilisée dans un procédé de décoloration des fibres kératiniques humaines et en particulier des cheveux.
Le procédé de décoloration selon l'invention comprend une étape d'application sur les fibres kératiniques d'une émulsion oxydante selon l'invention, cette émulsion comprenant de préférence de l'eau oxygénée en milieu alcalin après mélange extemporané.

Classiquement, une deuxième étape du procédé de décoloration selon l'invention est une étape de rinçage des fibres kératiniques.

Un autre objet de la présente invention est un procédé de déformation permanente des fibres kératiniques humaines et en particulier des cheveux, utilisant comme composition oxydante l'émulsion oxydante définie ci-dessus.
Selon ce procédé, on applique sur la fibre kératinique à traiter une composition réductrice, la fibre kératinique étant mise sous tension mécanique avant, pendant ou après ladite application, on rince éventuellement la fibre, on applique sur la fibre éventuellement rincée l'émulsion oxydante de la présente invention puis on rince éventuellement à nouveau la fibre.
La première étape de ce procédé consiste à appliquer sur les cheveux une composition réductrice. Cette application se fait mèche par mèche ou globalement.
La composition réductrice comprend au moins un agent réducteur, qui peut être en particulier choisi parmi l'acide thioglycolique, la cystéine, la cystéamine, le thioglycolate
de glycérol, l'acide thiolactique, ou les sels des acides thiolactique ou thioglycolique. L'habituelle étape de mise sous tension des cheveux sous une forme correspondant à la forme finale désirée pour ces derniers (boucles par exemple) peut être mise en oeuvre par tout moyen, mécanique notamment, approprié et connu en soi pour maintenir sous tension des cheveux, tels que par exemple rouleaux, bigoudis et analogues.
Les cheveux peuvent également être mis en forme sans l'aide de moyens extérieurs, simplement avec les doigts.
Avant de procéder à l'étape suivante facultative de rinçage, il convient, de manière classique, de laisser reposer pendant quelques minutes, généralement entre 5 minutes et une heure, de préférence entre 10 et 30 minutes, la chevelure sur laquelle a été appliquée la composition réductrice, et ceci de façon à bien laisser le temps au réducteur d'agir correctement sur les cheveux. Cette phase d'attente est effectuée de préférence à une température allant de 35 °C à 45 °C, en protégeant de préférence également les cheveux par un bonnet.
Dans la deuxième étape facultative de rinçage, les cheveux imprégnés de la composition réductrice sont rincés soigneusement par une composition aqueuse.
Puis, dans une troisième ,étape, on applique sur les cheveux ainsi rincés l'émulsion oxydante selon la présente invention, dans le but de fixer la nouvelle forme imposée aux cheveux.
Comme dans le cas de l'application de la composition réductrice, la chevelure sur laquelle a été appliquée l'émulsion oxydante est ensuite, de manière classique, laissée dans une phase de repos ou d'attente qui dure quelques minutes, généralement entre 3 et 30 minutes, de préférence entre 5 et 15 minutes.
Si la tension des cheveux était maintenue par des moyens extérieurs, on peut retirer de la chevelure ces derniers (rouleaux, bigoudis et analogues) avant ou après l'étape de fixation.
Enfin, dans la dernière étape du procédé selon l'invention, étape facultative également, les cheveux imprégnés de la composition oxydante sont rincés soigneusement, généralement à l'eau.

Les exemples suivants illustrent l'invention sans présenter un caractère limitatif.

### EXEMPLES

On a préparé les deux compositions oxydantes A et B suivantes.
(teneurs exprimées en grammes de Matière Active MA*)

| | **Composition A** de l'art antérieur | **Composition B** selon l'invention |
|---|---|---|
| Alcool cétylique | 3 | 3 |
| Laurylsulfate de sodium | 0,5 | 0,5 |
| Alcool oléïque glycérolé à 2 moles de glycérol | 0,45 | 0,45 |
| Alcool oléïque glycérolé à 4 moles de glycérol | 0,35 | 0,35 |
| Agent antimousse : Simethicone | 0,045 | 0,045 |
| Agent séquestrant : DTPA | 0,06 | 0,06 |
| Pyrophosphate tétrasodique, 10 H2O | 0,02 | 0,02 |
| Stannate de sodium, 6 H2O | 0,04 | 0,04 |
| Polymère amphiphile selon l'invention ** | | 0,05 |
| Eau oxygénée à 50% | 12 MA* | 12 MA* |
| Acide phosphorique en solution aqueuse à 85% q.s pH | 3 | 3 |
| Eau déminéralisée q.s.p | 100 | 100 |

| | | |
|---|---|---|
| ** Copolymère A.M.P.S. (80) / ester méthacrylique d'alcool en C₁₆-C₁₈ oxyéthyléné par 25 moles d'oxyde d'éthylène (20), réticulé par du TMPTA, préparé et décrit dans la demande de brevet français N° 2 818 543. | | |

Pendant sept semaines, on a suivi l'évolution de la texture / viscosité des 2 compositions A et B.
Pour quantifier l'évolution de la texture dans le temps, on a défini le paramètre Δv. évolution de la texture dans le temps : Δv = viscosité mesurée à l'instant t - viscosité mesurée 24 heures après fabrication.

Les viscosités ont été mesurées à l'aide d'un rhéomat, à la température de 25°C et au mobile 2. Elles on été exprimées en unités de déviation.

| | Moyenne sur 3 fabrications (1 mesure par fabrication) + écart-type | | | | |
|---|---|---|---|---|---|
| Temps après fabrication | 1 semaine | 2 semaines | 3 semaines | 4 semaines | 7 semaines |
| Δv(A) | 5,0 (1,2) | 10,0 (2,3) | 15,0 (2,6) | 18,0 (2,2) | -5,0 (2,1) |
| Δv(B) | 2,6 (0,9) | 2,6 (0,8) | 4,6 (1,2) | 6,0 (1,3) | 7,0 (1,2) |

Ces résultats démontrent que la composition B, conforme à l'invention, évolue significativement moins dans le temps que la composition A de l'art antérieur. En outre, on a constaté qu'elle présentait une meilleure stabilité et préservera donc mieux dans le temps ses qualités d'usage pour une utilisation en teinture, décoloration, ou déformation permanente des cheveux.
Après 7 semaines, la composition B selon l'invention reste également stable en eau oxygénée.

## Revendications

1. Emulsion crème oxydante, huile-dans-eau, destinée au traitement des matières kératiniques, **caractérisée par le fait qu'**elle comprend au moins un agent oxydant, au moins un alcool gras en (C₈-C₃₀), au moins un agent tensioactif non ionique et/ou anionique et au moins un polymère amphiphile comportant au moins un motif acide 2-acrylamido-2-méthylpropane-sulfonique sous forme libre ou partiellement ou totalement neutralisée de formule (I) suivante et au moins un motif hydrophobe comportant de 6 à 50 atomes de carbone, 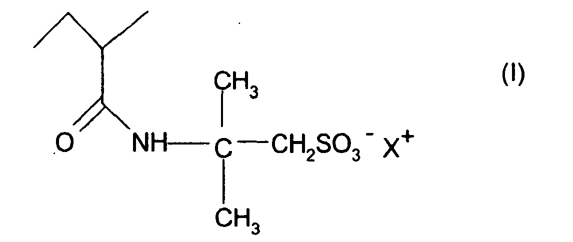 dans laquelle X⁺ est un proton, un cation de métal alcalin, un cation alcalino-terreux ou l'ion ammonium,

2. Emulsion selon la revendication 1, **caractérisée par le fait que** le motif hydrophobe du polymère amphiphile comporte de 12 à 22 atomes de carbone.

3. Emulsion selon l'une quelconque des revendications précédentes, **caractérisée par le fait que** les polymères amphiphiles ont un poids moléculaire moyen en nombre allant de 1000 à 20 000 000 g/mole, de préférence de 20 000 à 5 000 000 g/mole et plus particulièrement de 100 000 à 1 500 000 g/mole.

4. Emulsion selon l'une quelconque des revendications précédentes, **caractérisée par le fait qu'**une solution aqueuse à 1% en poids desdits polymères présente à la température de 25°C une viscosité mesurée au viscosimètre Brookfield, aiguille 7, allant de 20 000 mPas à 100 000 mPas.

5. Emulsion selon l'une quelconque des revendications précédentes, **caractérisée par le fait que** les polymères amphiphiles sont préparés par polymérisation radicalaire par précipitation dans le tert-butanol.

6. Emulsion selon l'une quelconque des revendications précédentes, **caractérisée par le fait que** les polymères amphiphiles sont réticulés ou non-réticulés et de préférence réticulés.

7. Emulsion selon la revendication 6, **caractérisée par le fait que** le ou les agents de réticulation sont choisis parmi les composés à polyinsaturation oléfinique.

8. Emulsion selon la revendication 7, **caractérisée par le fait que** le ou les agents de réticulation sont choisis parmi le méthylène-bis-acrylamide, le méthacrylate d'allyle ou le triméthylol propane triacrylate (TMPTA).

9. Emulsion selon l'une quelconque des revendications précédentes, **caractérisée par le fait que** le motif hydrophobe est choisi parmi les acrylates ou les acrylamides de formule (II) suivante : 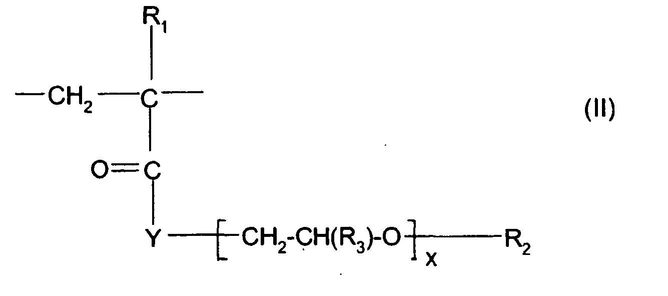 dans laquelle R₁ et R₃, identiques ou différents, désignent un atome d'hydrogène ou un radical alkyle linéaire ou ramifié en C₁-C₆ (de préférence méthyle) ; Y désigne O ou NH ; R₂ désigne un radical hydrocarboné hydrophobe comportant de 6 à 50 atomes de carbone et plus préférentiellement de 12 à 22 atomes de carbone; x désigne un nombre de moles d'oxyde d'alkylène et varie de 0 à 100.

10. Emulsion selon la revendication 9, **caractérisée par le fait que** le radical hydrophobe R₂ est choisi parmi les radicaux alkyles en C₆-C₁₈, linéaires, ramifiés ou cycliques ; les radicaux alkylperfluorés en C₆-C₁₈ ; le radical cholestéryle ou un ester de cholestérol ; les groupes polycycliques aromatiques.

11. Emulsion selon l'une quelconque des revendications 9 ou 10 **caractérisée par le fait que** le motif de formule (II) comporte en plus au moins un motif oxyde d'alkylène (x ≥ 1).

12. Emulsion selon l'une quelconque des revendications 9 à 11, **caractérisée par le fait que** le motif de formule (II) comporte en plus au moins une chaîne polyoxyalkylénée.

13. Emulsion selon la revendication 12, **caractérisée par le fait que** la chaîne polyoxyalkylénée est constituée de motifs oxyde d'éthylène et/ou de motifs oxyde de propylène.

14. Emulsion selon la revendication 13, **caractérisée par le fait que** la chaîne polyoxyalkylénée est constituée uniquement de motifs oxyde d'éthylène.

15. Emulsion selon l'une quelconque des revendications 9 à 14, **caractérisée par le fait que** le nombre de motifs oxyalkylénés varie de 3 à 100, de préférence de 3 à 50 et plus particulièrement de 7 à 25.

16. Emulsion selon l'une quelconque des revendications 9 à 10, **caractérisée par le fait que** le polymère amphiphile d'AMPS est choisi parmi :
- les copolymères réticulés ou non réticulés, neutralisés ou non, comportant de 15 à 60% en poids de motifs AMPS et de 40 à 85% en poids de motifs (C₈-C₁₆)alkyl(méth)acrylamide ou de motifs (C₈-C₁₆)alkyl(meth)acrylate, par rapport au polymère ;
- les terpolymères comportant de 10 à 90% en mole de motifs acrylamide, de 0,1 à 10% en mole de motifs AMPS et de 5 à 80% en mole de motifs n-(C₆-₁₈)alkylacrylamide, par rapport au polymère.

17. Emulsion selon l'une quelconque des revendications 9 à 10, **caractérisée par le fait que** le polymère amphiphile d'AMPS est choisi parmi :
- les copolymères non réticulés et réticulés d'AMPS partiellement ou totalement neutralisé et de méthacrylate de n-dodécyle.
- les copolymères non réticulés et réticulés d'AMPS partiellement ou totalement neutralisé et de n-dodécylméthacrylamide.

18. Emulsion selon l'une quelconque des revendications 9 à 15, **caractérisée par le fait que** le polymère amphiphile d'AMPS est choisi parmi les copolymères constitués de motifs acide 2-acrylamido-2-méthylpropane-sulfonique (AMPS) de formule (I) et de motifs de formule (III) suivante : 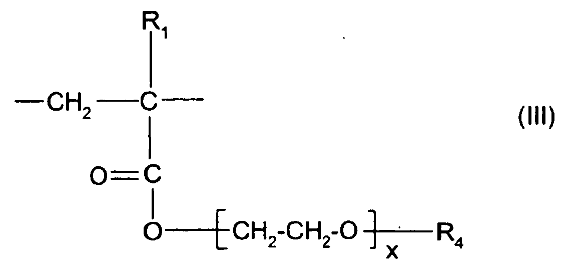 dans laquelle x désigne un nombre entier variant de 3 à 100, de préférence de 5 à 80 et plus préférentiellement de 7 à 25 ; R₁ a la même signification que celle indiquée ci-dessus dans la formule (II) et R₄ désigne un alkyle linéaire ou ramifié en C₆-C₂₂ et plus préférentiellement en C₁₀-C₂₂.

19. Emulsion selon la revendication 18, **caractérisée par le fait que** x = 25 , R₁ est méthyle et R₄ est en C₁₆-C₁₈ ou C₂₂.

20. Emulsion selon la revendication 9 ou 18, **caractérisée par le fait que** la proportion molaire en % des motifs de formule (II) ou des motifs de formule (III) dans les polymères varie de 50,1 à 99,9%.

21. Emulsion selon la revendication 9 ou 18, **caractérisée par le fait que** la proportion molaire en % des motifs de formule (II) ou des motifs de formule (III) dans les polymères varie de 0,1 à 50%.

22. Emulsion selon l'une quelconque des revendications précédentes, **caractérisée par le fait que** les polymères amphiphiles sont présents dans des concentrations allant de 0,01 à 10% en poids, plus préférentiellement de 0,01 à 5% en poids, encore plus préférentiellement de 0,01 à 2% en poids, par rapport au poids total de l'émulsion.

23. Emulsion selon l'une quelconque des revendications précédentes, **caractérisée par le fait que** l'agent oxydant est choisi parmi le peroxyde d'hydrogène, le peroxyde d'urée, les bromates ou ferricyanures de métaux alcalins, les persels ou leurs mélanges, les enzymes d'oxydoréduction telles que les laccases, les peroxydases et les oxydoréductases à 2 électrons, le cas échéant en présence de leur donneur ou cofacteur respectif.

24. Emulsion selon l'une quelconque des revendications précédentes, **caractérisée par le fait que** l'agent oxydant est le peroxyde d'hydrogène.

25. Emulsion selon la revendication 24, **caractérisée par le fait que** la concentration en peroxyde d'hydrogène varie de 0,15 à 12% et de préférence de 0,6 à 9% en poids par rapport au poids total de l'émulsion.

26. Emulsion selon la revendication 24, **caractérisée par le fait que** l'agent oxydant est l'eau oxygénée.

27. Emulsion selon l'une quelconque des revendications précédentes, **caractérisée par le fait que** la concentration en agents oxydants varie de 0,1 à 25% en poids par rapport au poids total de l'émulsion.

28. Emulsion selon l'une quelconque des revendications précédentes, **caractérisée par le fait que** son pH varie de 1 à 6, et de préférence de 2 à 4.

29. Emulsion selon l'une quelconque des revendications précédentes, **caractérisée par le fait que** le ou les alcools gras sont en C₁₂-C₂₂.

30. Emulsion selon la revendication 29, **caractérisée par le fait que** l'alcool gras est l'alcool cétylique.

31. Emulsion selon l'une quelconque des revendications précédentes, **caractérisée par le fait que** le ou les alcools gras sont présents dans des concentrations allant de 0,1 à 30% en poids, plus préférentiellement de 0,5 à 15% en poids, par rapport au poids total de l'émulsion.

32. Emulsion selon l'une quelconque des revendications précédentes, **caractérisée par le fait que** le ou les agents tensioactifs anioniques sont choisis parmi les alkylsulfates les alkyléthersulfates et les α-oléfine-sulfonates.

33. Emulsion selon l'une quelconque des revendications 1 à 31, **caractérisée par le fait que** le ou les agents tensioactifs non ioniques sont choisis parmi les alcools gras glycérolés.

34. Emulsion selon l'une quelconque des revendications précédentes, **caractérisée par le fait que** le ou les agents tensioactifs sont choisis parmi les mélanges de tensioactifs non ioniques et anioniques.

35. Emulsion selon l'une quelconque des revendications précédentes, **caractérisée par le fait que** le ou les agents tensioactifs sont présents dans des concentrations allant de 0,1 à 30% en poids, plus préférentiellement de 0,5 à 15% en poids, par rapport au poids total de l'émulsion.

36. Procédé de teinture d'oxydation des fibres kératiniques humaines et en particulier les cheveux mettant en oeuvre une composition colorante comprenant dans un support approprié pour la teinture des fibres kératiniques au moins un colorant d'oxydation et une émulsion oxydante telle que définie dans l'une quelconque des revendications 1 à 35.

37. Procédé de teinture selon la revendication 36 selon lequel on mélange, au moment de l'emploi, la composition colorante avec l'émulsion oxydante ; le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

38. Procédé de teinture selon la revendication 36 selon lequel on applique séquentiellement la composition colorante et l'émulsion oxydante, avec ou sans rinçage intermédiaire.

39. Procédé de traitement des fibres kératiniques humaines et en particulier les cheveux, en vue d'obtenir une déformation permanente de ces dernières, en particulier sous la forme de cheveux permanentés, ce procédé comprenant les étapes suivantes : (i) on applique sur la matière kératinique à traiter une composition réductrice, la fibre kératinique étant mise sous tension mécanique avant, pendant, ou après ladite application, (ii) on rince éventuellement la fibre kératinique, (iii) on applique sur la fibre kératinique éventuellement rincée une émulsion oxydante telle que définie à l'une des revendications 1 à 35, (iv) on rince éventuellement à nouveau la fibre kératinique.

40. Procédé de décoloration des fibres kératiniques humaines et en particulier les cheveux, comprenant les étapes suivantes : i) on applique sur la fibre kératinique une émulsion oxydante selon l'une quelconque des revendications 1 à 35, ii) on rince la fibre kératinique ainsi traitée.

41. Utilisation d'un polymère amphiphile tel que défini à l'une quelconque des revendications 1 à 21 pour stabiliser la viscosité d'une émulsion oxydante huile-dans eau comprenant au moins un alcool gras et au moins un agent tensioactif non ionique et/ou anionique.

## Claims

1. Oxidizing cream oil-in-water emulsion for treating keratin materials, **characterized in that** it comprises at least one oxidizing agent, at least one (C₈-C₃₀) fatty alcohol, at least one nonionic and/or anionic surfactant and at least one amphiphilic polymer comprising at least one 2-acrylamido-2-methylpropanesulphonic acid unit in free form or partially or totally neutralized form of formula (I) below and at least one hydrophobic unit containing from 6 to 50 carbon atoms, 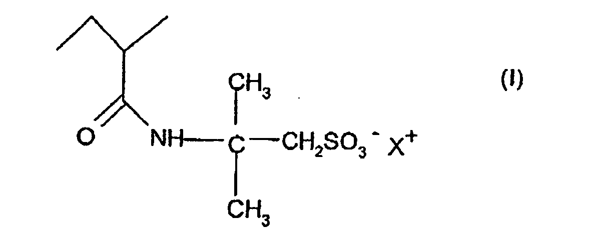 in which X⁺ is a proton, an alkali metal cation, an alkaline-earth metal cation or an ammonium ion.

2. Emulsion according to Claim 1, **characterized in that** the hydrophobic unit of the amphiphilic polymer contains from 12 to 22 carbon atoms.

3. Emulsion according to either of the preceding claims, **characterized in that** the amphiphilic polymers have a number-average molecular weight ranging from 1 000 to 20 000 000 g/mol, preferably from 20 000 to 5 000 000 g/mol and more particularly from 100 000 to 1 500 000 g/mol.

4. Emulsion according to any one of the preceding claims, **characterized in that** an aqueous 1% by weight solution of the said polymers has, at a temperature of 25°C, a viscosity measured using a Brookfield viscometer, No. 7 needle, ranging from 20 000 mPa.s to 100 000 mPa.s.

5. Emulsion according to any one of the preceding claims, **characterized in that** the amphiphilic polymers are prepared by free-radical polymerization by precipitation in tert-butanol.

6. Emulsion according to any one of the preceding claims, **characterized in that** the amphiphilic polymers are crosslinked or non-crosslinked, but preferably crosslinked.

7. Emulsion according to Claim 6, **characterized in that** the crosslinking agent(s) is(are) chosen from polyolefinically unsaturated compounds.

8. Emulsion according to Claim 7, **characterized in that** the crosslinking agent(s) is (are) chosen from methylenebisacrylamide, allyl methacrylate and trimethylolpropane triacrylate (TMPTA).

9. Emulsion according to any one of the preceding claims, **characterized in that** the hydrophobic unit is chosen from acrylates and acrylamides of formula (II) below: 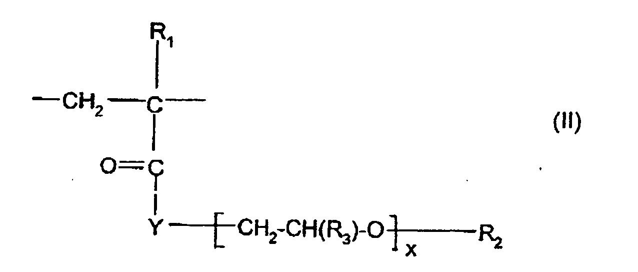 in which R₁ and R₃, which may be identical or different, denote a hydrogen atom or a linear or branched C₁-C₆ alkyl radical (preferably methyl); Y denotes O or NH; R₂ denotes a hydrophobic hydrocarbon-based radical containing from 6 to 50 carbon atoms and more preferably from 12 to 22 carbon atoms; x denotes a number of moles of alkylene oxide and ranges from 0 to 100.

10. Emulsion according to Claim 9, **characterized in that** the hydrophobic radical R₂ is chosen from linear, branched or cyclic C₆-C₁₈ alkyl radicals; C₆-C₁₈ perfluoroalkyl radicals; the cholesteryl radical or a cholesterol ester; aromatic polycyclic groups.

11. Emulsion according to either of Claims 9 and 10, **characterized in that** the unit of formula (II) also comprises at least one alkylene oxide unit (x ≥ 1).

12. Emulsion according to any one of Claims 9 to 11, **characterized in that** the unit of formula (II) also comprises at least one polyoxyalkylenated chain.

13. Emulsion according to Claim 12, **characterized in that** the polyoxyalkylenated chain consists of ethylene oxide units and/or of propylene oxide units.

14. Emulsion according to Claim 13, **characterized in that** the polyoxyalkylenated chain consists solely of ethylene oxide units.

15. Emulsion according to any one of Claims 9 to 14, **characterized in that** the number of oxyalkylene units ranges from 3 to 100, preferably from 3 to 50 and more particularly from 7 to 25.

16. Emulsion according to either of Claims 9 and 10, **characterized in that** the amphiphilic AMPS polymer is chosen from:
- crosslinked or noncrosslinked, neutralized or non-neutralized copolymers comprising from 15% to 60% by weight of AMPS units and from 40% to 85% by weight of (C₈-C₁₆)alkyl(meth)acrylamide units or of (C₈-C₁₆)alkyl (meth)acrylate units, relative to the polymer;
- terpolymers comprising from 10 mol% to 90 mol% of acrylamide units, from 0.1 mol% to 10 mol% of AMPS units and from 5 mol% to 80 mol% of n-(C₆-C₁₈)alkylacrylamide units, relative to the polymer.

17. Emulsion according to either of Claims 9 and 10, **characterized in that** the amphiphilic AMPS polymer is chosen from:
- non-crosslinked and crosslinked copolymers of partially or totally neutralized AMPS and of n-dodecyl methacrylate;
- non-crosslinked and crosslinked copolymers of partially or totally neutralized AMPS and of n-dodecylmethacrylamide.

18. Emulsion according to any one of Claims 9 to 15, **characterized in that** the amphiphilic AMPS polymer is chosen from copolymers consisting of 2-acrylamido-2-methylpropanesulphonic (AMPS) acid units of formula (I) and of units of formula (III) below: 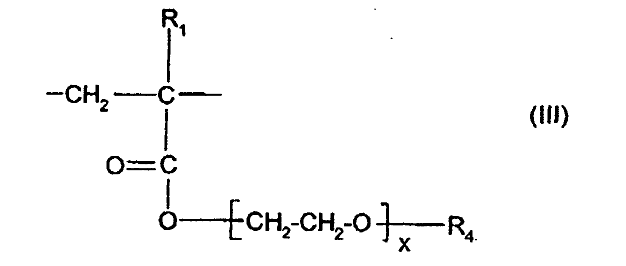 in which x denotes an integer ranging from 3 to 100, preferably from 5 to 80 and more preferably from 7 to 25; R₁ has the same meaning as that given above in formula (II) and R₄ denotes a linear or branched C₆-C₂₂ and more preferably C₁₀-C₂₂ alkyl.

19. Emulsion according to Claim 18, **characterized in that** x = 25, R₁ is methyl and R₄ is C₁₆-C₁₈ or C₂₂.

20. Emulsion according to Claim 9 or 18, **characterized in that** the molar percentage proportion of the units of formula (II) or of the units of formula (III) in the polymers ranges from 50.1% to 99.9%.

21. Emulsion according to Claim 9 or 18, **characterized in that** the molar percentage proportion of the units of formula (II) or of the units of formula (III) in the polymers ranges from 0.1% to 50%.

22. Emulsion according to any one of the preceding claims, **characterized in that** the amphiphilic polymers are present in concentrations ranging from 0.01% to 10% by weight, more preferably from 0.01% to 5% by weight and even more preferably from 0.01% to 2% by weight, relative to the total weight of the emulsion.

23. Emulsion according to any one of the preceding claims, **characterized in that** the oxidizing agent is chosen from hydrogen peroxide, urea peroxide, alkali metal bromates or ferricyanides, persalts or mixtures thereof, redox enzymes such as laccases, peroxidases and 2-electron oxidoreductases, where appropriate in the presence of the respective donor or co-factor thereof.

24. Emulsion according to any one of the preceding claims, **characterized in that** the oxidizing agent is hydrogen peroxide.

25. Emulsion according to Claim 24, **characterized in that** the hydrogen peroxide concentration ranges from 0.15% to 12% and preferably from 0.6% to 9% by weight relative to the total weight of the emulsion.

26. Emulsion according to Claim 24, **characterized in that** the oxidizing agent is aqueous hydrogen peroxide solution.

27. Emulsion according to any one of the preceding claims, **characterized in that** the oxidizing agent concentration ranges from 0.1% to 25% by weight relative to the total weight of the emulsion.

28. Emulsion according to any one of the preceding claims, **characterized in that** its pH ranges from 1 to 6 and preferably from 2 to 4.

29. Emulsion according to any one of the preceding claims, **characterized in that** the fatty alcohol(s) is (are) C₁₂-C₂₂.

30. Emulsion according to Claim 29, **characterized in that** the fatty alcohol is cetyl alcohol.

31. Emulsion according to any one of the preceding claims, **characterized in that** the fatty alcohol(s) is(are) present in concentrations ranging from 0.1% to 30% by weight and more preferably from 0.5% to 15% by weight relative to the total weight of the emulsion.

32. Emulsion according to any one of the preceding claims, **characterized in that** the anionic surfactant(s) is(are) chosen from alkyl sulphates, alkyl ether sulphates and α-olefin sulphonates.

33. Emulsion according to any one of Claims 1 to 31, **characterized in that** the nonionic surfactant(s) is(are) chosen from glycerolated fatty alcohols.

34. Emulsion according to any one of the preceding claims, **characterized in that** the surfactant(s) is (are) chosen from mixtures of nonionic and anionic surfactants.

35. Emulsion according to any one of the preceding claims, **characterized in that** the surfactant(s) is(are) present in concentrations ranging from 0.1% to 30% by weight and more preferably from 0.5% to 15% by weight relative to the total weight of the emulsion.

36. Process for the oxidation dyeing of human keratin fibres and in particular the hair using a dye composition comprising, in a support that is suitable for dyeing keratin fibres, at least one oxidation dye and an oxidizing emulsion as defined in any one of Claims 1 to 35.

37. Dyeing process according to Claim 36, according to which the dye composition is mixed, at the time of use, with the oxidizing emulsion; the mixture obtained is then applied to the keratin fibres and it is left in for 3 to 50 minutes approximately and preferably 5 to 30 minutes approximately, after which the fibres are rinsed, washed with shampoo, rinsed again and dried.

38. Dyeing process according to Claim 36, according to which the dye composition and the oxidizing emulsion are applied sequentially, with or without intermediate rinsing.

39. Process for treating human keratin fibres and in particular the hair in order to permanently reshape these fibres, in particular in the form of permanent-waved hair, this process comprising the following steps: (i) a reducing composition is applied to the keratin material to be treated, the keratin fibre being placed under mechanical tension before, during or after the said application, (ii) the keratin fibre is optionally rinsed, (iii) an oxidizing emulsion as defined in one of Claims 1 to 35 is applied to the optionally rinsed keratin fibre, (iv) the keratin fibre is optionally rinsed again.

40. Process for bleaching human keratin fibres and in particular the hair, comprising the following steps: i) an oxidizing emulsion according to any one of Claims 1 to 35 is applied to the keratin fibre, ii) the keratin fibre thus treated is rinsed.

41. Use of an amphiphilic polymer as defined in any one of Claims 1 to 21, to stabilize the viscosity of an oxidizing oil-in-water emulsion comprising at least one fatty alcohol and at least one nonionic and/or anionic surfactant.

## Patentansprüche

1. Oxidierende Öl-in-Wasser-Emulsion in Cremeform, die für die Behandlung von Keratinsubstanzen vorgesehen ist, **dadurch gekennzeichnet, dass** sie mindestens ein Oxidationsmittel, mindestens einen C₈₋₃₀-Fettalkohol, mindestens einen nichtionischen und/oder anionischen grenzflächenaktiven Stoff und mindestens ein amphiphiles Polymer enthält, das mindestens eine 2-Acrylamido-2-methylpropan-sulfonsäureeinheit der folgenden Formel (I) in freier Form oder ganz oder teilweise neutralisiert und mindestens eine hydrophobe Einheit mit 6 bis 50 Kohlenstoffatomen enthält: 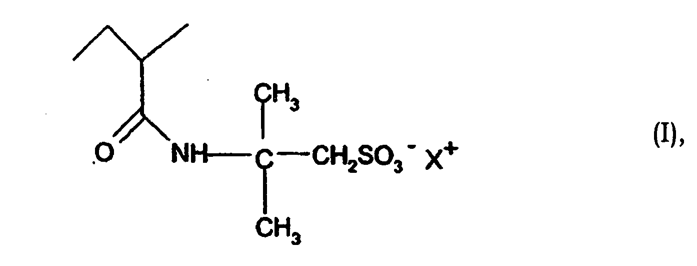 worin X⁺ ein Proton bedeutet, ein Alkalimetallkation, ein Erdalkalimetallkation oder das Ammoniumion.

2. Emulsion nach Anspruch 1, **dadurch gekennzeichnet, dass** die hydrophobe Einheit des amphiphilen Polymers 12 bis 22 Kohlenstoffatome aufweist.

3. Emulsion nach einem der vorhergehenden Ansprüche, **dadurch gekennzeichnet, dass** die amphiphilen Polymere eine zahlenmittlere Molmasse von 1 000 bis 20 000 000 g/mol, vorzugsweise 20 000 bis 5 000 000 g/mol und insbesondere 100 000 bis 1 500 000 g/mol aufweisen.

4. Emulsion nach einem der vorhergehenden Ansprüche, **dadurch gekennzeichnet, dass** eine wässrige Lösung mit 1 Gew.-% der Polymere bei einer Umgebungstemperatur von 25 °C eine mit dem Brookfield-Viskosimeter, Nadel 7, gemessene Viskosität von 20 000 bis 100 000 mPa aufweist.

5. Emulsion nach einem der vorhergehenden Ansprüche, **dadurch gekennzeichnet, dass** die amphiphilen Polymere durch radikalische Polymerisation unter Ausfällen in t-Butanol hergestellt werden.

6. Emulsion nach einem der vorhergehenden Ansprüche, **dadurch gekennzeichnet, dass** die amphiphilen Polymere vernetzt oder nicht vernetzt und vorzugsweise vernetzt sind.

7. Emulsion nach Anspruch 6, **dadurch gekennzeichnet, dass** das oder die Vernetzungsmittel unter den Verbindungen mit mehreren olefinisch ungesättigten Bindungen ausgewählt sind.

8. Emulsion nach Anspruch 7, **dadurch gekennzeichnet, dass** das oder die Vernetzungsmittel unter Methylen-bis-acrylamid, Allylmethacrylat oder Trimethylolpropantriacrylat (TMPTA) ausgewählt sind.

9. Emulsion nach einem der vorhergehenden Ansprüche, **dadurch gekennzeichnet, dass** die hydrophobe Einheit unter den Acrylaten oder Acrylamiden der folgenden Formel (II) ausgewählt ist: 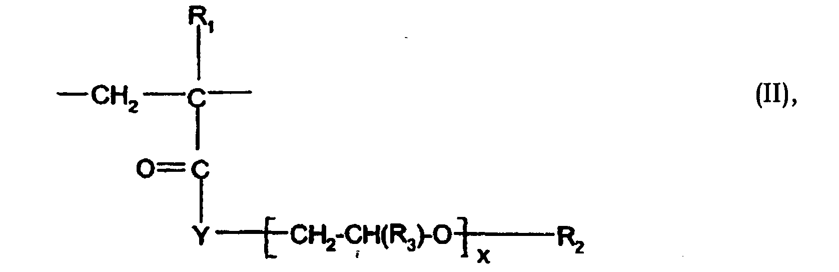 worin R₁ und R₃, die gleich oder verschieden sind, ein Wasserstoffatom oder eine geradkettige oder verzweigte C₁₋₆-Alkylgruppe (vorzugsweise Methyl) bedeuten; Y O oder NH ist; R₂ eine hydrophobe Kohlenwasserstoffgruppe mit 6 bis 50 Kohlenstoffatomen und vorzugsweise 12 bis 22 Kohlenstoffatomen bedeutet; und x eine Alkylenoxidmolzahl bedeutet und im Bereich von 0 bis 100 liegt.

10. Emulsion nach Anspruch 9, **dadurch gekennzeichnet, dass** die hydrophobe Gruppe R₂ unter den geradkettigen, verzweigten oder cyclischen C₆₋₁₈-Alkylgruppen, C₆₋₁₈-Alkylperfluorgruppen; der Cholesterylgruppe oder einem Cholesterinester; und aromatischen polycyclischen Gruppen ausgewählt ist.

11. Emulsion nach einem der Ansprüche 9 oder 10, **dadurch gekennzeichnet, dass** die Einheit der Formel (II) ferner mindestens eine Alkylenoxideinheit (x ≥ 1) aufweist.

12. Emulsion nach einem der Ansprüche 9 bis 11, **dadurch gekennzeichnet, dass** die Einheit der Formel (II) ferner mindestens eine Polyoxyalkylenkette aufweist.

13. Emulsion nach Anspruch 12, **dadurch gekennzeichnet, dass** die Polyoxyalkylenkette aus Ethylenoxideinheiten und/oder Propylenoxideinheiten besteht.

14. Emulsion nach Anspruch 13, **dadurch gekennzeichnet, dass** die Polyoxyalkylenkette ausschließlich aus Ethylenoxideinheiten besteht.

15. Emulsion nach einem der Ansprüche 9 bis 14, **dadurch gekennzeichnet, dass** die Anzahl der Oxyalkyleneinheiten im Bereich von 3 bis 100, vorzugsweise 3 bis 50 und insbesondere 7 bis 25 liegt.

16. Emulsion nach einem der Ansprüche 9 bis 10, **dadurch gekennzeichnet, dass** das amphiphile AMPS Polymer ausgewählt ist unter:
- vernetzten oder nicht vernetzten, neutralisierten oder nicht neutralisierten Copolymeren, die 15 bis 60 Gew.-% AMPS-Einheiten und 40 bis 85 Gew.-% C₈₋₁₆-Alkyl(meth)acrylamidEinheiten oder C₈₋₁₆-Alkyl(meth)acrylat-Einheiten, bezogen auf das Polymer, enthalten;
- Terpolymeren, die 10 bis 90 Mol-% Acrylamideinheiten, 0,1 bis 10 Mol-% AMPS-Einheiten und 5 bis 80 Mol-% n-(C₆₋₁₈)Alkylacrylamid-Einheiten, bezogen auf das Polymer, enthalten.

17. Emulsion nach einem der Ansprüche 9 bis 10, **dadurch gekennzeichnet, dass** das amphiphile AMPS-Polymer ausgewählt ist unter:
- nicht vernetzten und vernetzten Copolymeren von ganz oder teilweise neutralisierter AMPS und n-Dodecylmethacrylat,
- nicht vernetzten und vernetzten Copolymeren von ganz oder teilweise neutralisierter AMPS und n-Dodecylmethacrylamid.

18. Emulsion nach einem der Ansprüche 9 bis 15, **dadurch gekennzeichnet, dass** das amphiphile AMPS-Polymer unter den Copolymeren ausgewählt ist, die aus 2-Acrylamido-2-methylpropansulfonsäure-Einheiten (AMPS) der Formel (I) und Einheiten der folgenden Formel (III) bestehen: 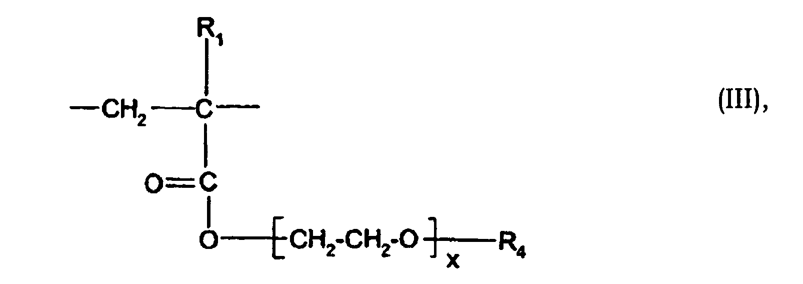 worin x eine ganze Zahl von 3 bis 100, vorzugsweise 5 bis 80 und noch bevorzugter 7 bis 25 bedeutet; R₁ die oben für die Formel (II) angegebenen Bedeutungen aufweist und R₄ eine geradkettige oder verzweigte C₆₋₂₂-Alkylgruppe und noch bevorzugter C₁₀₋₂₂-Alkylgruppe bedeutet.

19. Emulsion nach Anspruch 18, **dadurch gekennzeichnet, dass** x = 25, R₁ Methyl bedeutet und R₄ 16 bis 18 oder 22 Kohlenstoffatome aufweist.

20. Emulsion nach Anspruch 9 oder 18, **dadurch gekennzeichnet, dass** der molare prozentuale Anteil der Einheiten der Formel (II) oder der Einheiten der Formel (III) in den Polymeren im Bereich von 50,1 bis 99,9 % liegt.

21. Emulsion nach Anspruch 9 oder 18, **dadurch gekennzeichnet, dass** der prozentuale molare Anteil der Einheiten der Formel (II) oder der Einheiten der Formel (III) in den Polymeren im Bereich von 0, 1 bis 50 % liegt.

22. Emulsion nach einem der vorhergehenden Ansprüche, **dadurch gekennzeichnet, dass** die amphiphilen Polymere in Konzentrationen von 0,01 bis 10 Gew.-%, bevorzugt 0,01 bis 5 Gew.-% und noch bevorzugter 0,01 bis 2 Gew.-%, bezogen auf das Gesamtgewicht der Emulsion, vorliegen.

23. Emulsion nach einem der vorhergehenden Ansprüche, **dadurch gekennzeichnet, dass** das Oxidationsmittel unter Wasserstoffperoxid, Harnstoffperoxid, Alkalimetallbromaten, Alkalimetallferricyaniden, Salzen von Persäuren oder deren Gemischen, Redoxenzymen, wie Laccasen, Peroxidasen und Oxidoreductasen (2 Elektronen) gegebenenfalls in Gegenwart ihres jeweiligen Donors oder Cofaktors ausgewählt ist.

24. Emulsion nach einem der vorhergehenden Ansprüche, **dadurch gekennzeichnet, dass** das Oxidationsmittel das Wasserstoffperoxid ist.

25. Emulsion nach Anspruch 24, **dadurch gekennzeichnet, dass** die Wasserstoffperoxidkonzentration im Bereich von 0,15 bis 12 % und vorzugsweise 0,6 bis 9 Gew.-%, bezogen auf das Gesamtgewicht der Emulsion, liegt.

26. Emulsion nach Anspruch 24, **dadurch gekennzeichnet, dass** das Oxidationsmittel eine wässrigen Wasserstoffperoxidlösung ist.

27. Emulsion nach einem der vorhergehenden Ansprüche, **dadurch gekennzeichnet, dass** die Konzentration der Oxidationsmittel im Bereich von 0,1 bis 25 Gew.-%, bezogen auf das Gesamtgewicht der Emulsion, liegt.

28. Emulsion nach einem der vorhergehenden Ansprüche, **dadurch gekennzeichnet, dass** ihr pH-Wert im Bereich von 1 bis 6 und vorzugsweise 2 bis 4 liegt.

29. Emulsion nach einem der vorhergehenden Ansprüche, **dadurch gekennzeichnet, dass** der oder die Fettalkohol(e) 12 bis 22 Kohlenstoffatome aufweisen.

30. Emulsion nach Anspruch 29, **dadurch gekennzeichnet, dass** der Fettalkohol der Cetylalkohol ist.

31. Emulsion nach einem der vorhergehenden Ansprüche, **dadurch gekennzeichnet, dass** der oder die Fettalkohole in Konzentrationen von 0,1 bis 30 Gew.-% und vorzugsweise 0,5 bis 15 Gew.-%, bezogen auf das Gesamtgewicht der Emulsion, enthalten sind.

32. Emulsion nach einem der vorhergehenden Ansprüche, **dadurch gekennzeichnet, dass** der oder die anionischen grenzflächenaktiven Stoffe unter den Alkylsulfaten, Alkylethersulfaten und α-Olefinsulfonaten ausgewählt sind.

33. Emulsion nach einem der Ansprüche 1 bis 31, **dadurch gekennzeichnet, dass** der oder die nichtionischen grenzflächenaktiven Stoffe unter den mit Glycerin veretherten Fettalkoholen ausgewählt sind.

34. Emulsion nach einem der vorhergehenden Ansprüche, **dadurch gekennzeichnet, dass** der oder die grenzflächenaktive(n) Stoff(e) unter den Gemischen von nichtionischen grenzflächenaktiven Stoffen und anionischen grenzflächenaktiven Stoffen ausgewählt sind.

35. Emulsion nach einem der vorhergehenden Ansprüche, **dadurch gekennzeichnet, dass** der oder die grenzflächenaktive(n) Stoff(e) in Konzentrationen von 0,1 bis 30 Gew.-% und vorzugsweise 0,5 bis 15 Gew.-%, bezogen auf das Gesamtgewicht der Emulsion, enthalten sind.

36. Verfahren zum oxidativen Färben von menschlichen Keratinfasern und insbesondere Haaren unter Verwendung einer Farbmittelzusammensetzung, die in einem zum Färben von Keratinfasern geeigneten Träger mindestens einen Oxidationsfarbstoff und eine oxidierende Emulsion nach einem der Ansprüche 1 bis 35 enthält.

37. Verfahren zum Färben nach Anspruch 36, wobei die Farbmittelzusammensetzung bei der Anwendung mit der oxidierenden Emulsion vermischt wird; das erhaltene Gemisch dann auf die Keratinfasern aufgetragen und etwa 3 bis 50 Minuten und vorzugsweise etwa 5 bis 30 Minuten einwirken gelassen wird, wonach gespült, mit Haarwaschmittel gewaschen, nochmals gespült und getrocknet wird.

38. Verfahren zum Färben nach Anspruch 36, wobei die Farbmittelzusammensetzung und die oxidierende Emulsion mit oder ohne zwischenzeitliches Spülen nacheinander aufgebracht werden.

39. Verfahren zur Behandlung von menschlichen Keratinfasern und insbesondere Haaren, um diese permanent zu verformen, insbesondere in der Form von dauergewellten Haaren, wobei das Verfahren die folgenden Schritte umfasst: (i) auf die zu behandelnde Keratinsubstanz wird eine reduzierende Zusammensetzung aufgetragen, wobei die Keratinfasern vor, während oder nach dem Aufbringen mechanisch unter Spannung gesetzt werden; (ii) die Keratinfasern werden gegebenenfalls gespült, (iii) auf die Keratinfasern, die gegebenenfalls gespült wurden, wird eine oxidierende Emulsion nach einem der Ansprüche 1 bis 35 aufgetragen und (iv) die Keratinfasern werden gegebenenfalls nochmals gespült.

40. Verfahren zum Entfärben von menschlichen Keratinfasern und insbesondere Haaren, das die folgenden Schritte umfasst: (i) auf die Keratinfasern wird eine oxidierende Emulsion nach einem der Ansprüche 1 bis 35 aufgetragen und (ii) die so behandelten Keratinfasern werden gespült.

41. Verwendung eines amphiphilen Polymers nach einem der Ansprüche 1 bis 21 zur Stabilisierung der Viskosität einer oxidierenden Öl-in-Wasser-Emulsion, die mindestens einen Fettalkohol und mindestens einen nichtionischen und/oder anionischen grenzflächenaktiven Stoff enthält.
